# EUROPEAN PATENT SPECIFICATION

(11) **EP 0 307 402 B1**
(45) Date of publication and mention of the grant of the patent: **11.08.1993**
(21) Application number: 87903104.5
(22) Date of filing: 13.04.1987
(51) Int. Cl.: C12P 21/00, C12N 15/00, C12N 1/00, C07K 13/00, C07K 15/00, A61K 37/00

(54) **PRODUCTION OF M-CSF**
HERSTELLUNG VON M-CSF
PRODUCTION DE M-CSF

(30) Priority: 06.05.1986 US 860377; 11.12.1986 US 940362
(43) Date of publication of application: 22.03.1989
(73) Proprietor: GENETICS INSTITUTE, INC., Cambridge, Massachusetts 02140 (US)
(72) Inventor: CLARK, Steven, C., Winchester, MA 01890 (US); WONG, Gordon, G., Cambridge, MA 02138 (US)
(74) Representative: VOSSIUS & PARTNER
(86) International application number: PCT/US87/00835
(87) International publication number: WO 87/06954

(56) References cited:
- EP-A- 0 261 592
- SCIENCE, vol. 228, 17 May 1985, Washington, DC (US); WONG et al., pp. 810-815#
- SCIENCE, vol. 230, 18 October 1985, Washington, DC (US); KAWASAKI et al., pp. 291-296#
- JOURNAL OF BIOLOGICAL CHEMISTRY, vol. 257, 25 November 1982, Baltimore, MD (US); DAS et al., pp. 13679-13681#
- BLOOD, vol. 60, November 1982, London (GB); BURGESS et al., pp. 1219-1223#
- JOURNAL OF BIOLOGICAL CHEMISTRY, vol. 256, 10 August 1981, Baltimore, MD (US); HEWICK et al., pp. 7990-7997#
- PROCEEDINGS OF THE NATL. ACADEMY OF SCIENCE USA, vol. 76, June 1979, Washington, DC (US); STANLEY, pp. 2969-2973#
- NATURE, vol. 309, 28 June 1984, London (GB); GOUGH et al., pp. 763-767#
- THE EMBO JOURNAL, vol. 4, March 1985, Oxford (GB); GOUGH et al., pp. 645-653#
- MOLECULAR & CELLULAR BIOLOGY, vol. 2, November 1982, Washington, DC (US); KAUFMAN et al., pp. 1304-1319#

## Description

The present invention relates to a novel DNA sequence and recombinant DNA molecule to produce human M-CSF glycoprotein.

M-CSF or CSF-1 is a protein characterized by the ability to promote the survival, growth and differentiation of colonies of macrophages; hence the name, macrophage-colony stimulating factor [M-CSF]. M-CSF has been the topic of numerous articles describing assays for its detection in murine and human systems for a number of years. Similarly, methods for the purification of this protein from natural sources have been described. For example, PCT application WO86/04587 discloses a method for purifying human and murine CSF-1 proteins from natural sources by employing immunoaffinity chromatography and reverse phase high pressure chromatography. See, also, e.g. E. R. Stanley et al, J. Biol. Chem., 252:4305 (1977); S. K. Das et al, Blood, 58:630 (1981) and references cited in PCT application WO86/04587.

A cDNA sequence encoding a form of human CSF-1 has been reported by E. S. Kawasaki et al, Science, 230:291-296 (1985). Additionally, published PCT application WO86/04607 describes a method for producing the protein encoded by that cDNA sequence by recombinant methods. Further, cDNA sequences encoding CSFs and their expression have been reported by Wong et al., Science, 228 : 810-815 (1985); Gough et al., Haematol. Blood. Transfus., 29 : 380-384 (1985); Dunn et al., Cancer Cells 3/Growth Factors and Transformation, 227-234, CSH-Laboratory (1985); Ralph, Leukocytes and Host Defense, 235-242, Alan R. Liss, Inc. (1986); Lusis et al., Nature, 298 : 75-77 (1982); Gough et al., EMBO J., 4 : 645-653 (1985); US-A-4,438,032.

There remains a need in the art for the production of other human CSF-1 or M-CSF proteins which mimic naturally-occurring human urinary M-CSF for therapeutic treatments of hematopoietic disorders.

As one aspect of the present invention, there has been surprisingly discovered a novel method for producing a previously unidentified human M-CSF protein substantially free from association with other human proteins. The method comprises culturing a suitable cell transformed with a novel cDNA sequence which encodes an M-CSF protein which differs significantly from the sequences described in the prior art. This novel sequence encodes a protein exhibiting biological and biochemical characteristics of natural human M-CSF and characterized by containing the same or substantially the same peptide sequence as amino acid #1 through amino acid #223, depicted in Fig. I.

Fig. I depicts the complete 4kb DNA sequence which codes on expression for the M-CSF protein. This sequence contains a long open translational reading frame of 1662 nucleotides, encoding a 554 amino acid polypeptide. Three portions, i.e. from nucleotide #1 to #415, from nucleotide #419 to #689 and from nucleotide #1584 to #1823, are also present in the Kawasaki, et. al., supra, sequence. However, nucleotides #416 to #418 and #690 to #1583 represent novel sequences which do not disrupt the reading frame of the coding sequence. The protein coding region of the 4kb sequence extends from nucleotide #146 (the adenine in the methionine codon) to nucleotide #1807 which is followed by a TAG stop codon. There are four potential asparagine-linked glycosylation sites illustrated by the characteristic sequences, Asn-X-Ser or Asn-X-Thr. The remaining 2200 nucleotides of the 3' non-coding sequence of the 4kb region may have a regulatory role in transcription in the natural host. The 3' end of the sequence also contains an AT-rich segment including several repeats of the sequence ATTTA, which is believed to be related to the RNA message stability [See, G. Shaw and R. Kamen, Cell, 46(5):659-677 (1986)].

The cDNA sequence used in the method of the present invention is in operative association with an expression control sequence. The cDNA sequence contains a previously unidentified novel sequence -- the identical nucleotide sequence or substantially the same nucleotide sequence as nucleotide #242 through nucleotide #910, or nucleotide #242 through nucleotide #1583, as depicted in Fig. I. One such novel and preferred cDNA sequence includes the complete nucleotide sequence of Fig. I. Additionally, allelic variants (or isozymes) of one nucleotide and corresponding peptide sequences of Fig. I and variations in the nucleotide sequence resulting from the degeneracy of the genetic code are also encompassed in the invention where they encode a polypeptide having M-CSF activity.

Also part of the present invention is a process for producing M-CSF in which the cell to be cultured is transformed with a cDNA sequence encoding M-CSF protein characterized by containing a peptide sequence the same or substantially the same as that of amino acid #150 through amino acid #223. The cDNA sequence for use in this method includes a nucleotide sequence substantially the same, or the same, as the nucleotide sequence from nucleotide #689 through nucleotide #910 as depicted in Fig. I. This invention encompasses the production of all proteins having M-CSF activity which are characterized by the same or substantially the same partial peptide sequence encoded by this novel nucleotide sequence.

The transformed cell employed in the method also contains suitable expression control sequences in operative association with the M-CSF DNA coding sequence. Suitable cells or cell lines may be mammalian cells, such as Chinese hamster ovary (CHO) cells. The selection of suitable mammalian host cells and methods for transformation, culture, amplification, screening and product production and purification are known in the art. See, e.g. Gething and Sambrook, Nature, 293:620-625 (1981), or alternatively, Kaufman et al, Mol. Cell. Biol., 5(7):1750-1759 (1985) or Howley et al, U.S. Patent 4,419,446. Another suitable mammalian cell line, which is described in the accompanying examples, is the monkey COS-1 cell line. A similarly useful mammalian cell line is the CV-1 cell line.

Also suitable for use in the present invention are bacterial cells. For example, the various strains of E. coli are well-known as host cells in the field of biotechnology. Various strains of B. subtilis may also be employed in this method. Many strains of yeast cells known to those skilled in the art are also available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references cited therein.

Another aspect of the present invention provides vectors for use in the method of expression of this M-CSF protein which contain the same, or substantially the same, nucleotide sequence as the sequence of nucleotide #242 through #910, or #689 through #910, or #242 through #1583, or #689 through #1583 or the entire nucleotide sequence, each as depicted in Fig. I. Preferably the vectors contain the full DNA sequence recited in Fig. I. The vectors also contain appropriate expression control sequences permitting expression of the M-CSF DNA sequence. Alternatively, vectors incorporating modified or naturally occurring allelic sequences as described herein are also embodiments of the present invention and useful in the production of M-CSF. The vector may be employed in the method of transforming cell lines and contain selected regulatory sequences in operative association with the DNA coding sequences of the invention which are capable of directing the replication and expression thereof in selected host cells. Useful regulatory sequences for such vectors are known to one of skill in the art and may be selected depending upon the selected host cells. Such selection is routine and does not form part of the present invention.

Still another aspect of the present invention is the DNA sequence of Fig. I and its allelic variations. The approximately 4kb DNA sequence of Fig. I is harbored in plasmid p3ACSF-69 in E. coli HB 101, which was deposited in the American Type Culture Collection, 12301 Parklawn Dr., Rockville, MD on April 16, 1986 and given accession number ATCC 67092. The sequence, transfected into appropriate host cells, codes on expression for the M-CSF protein which demonstrates macrophage colony stimulating activity in in vitro mouse and human bone marrow assays.

Slight variations in the 4kb sequence of Fig. I which are caused by point mutations and the like should not change the functional protein for which the sequence codes on expression. Therefore, such minor variations in sequence, including those due to the degeneracies of the genetic code are encompassed in the invention. Nucleotide modifications can also be deliberately engineered into the DNA sequence employed in this method, which modifications can be made by one skilled in the art using known techniques. Such modification can cause the deletion, insertion or substitution of amino acids. For example, the replacement of one or more of the cysteine residues in the coding sequence can eliminate a corresponding disulfide bridge. Additionally, the substitution, insertion or deletion of an amino acid at one or more of the tripeptide asparagine-linked glycosylation recognition sites can result in non-glycosylation at that site. Mutagenic techniques for such replacement or deletion are well known to one skilled in the art. [See, United States patent 4,518,584]. Vectors incorporating these modified sequences are also embodiments of the present invention and useful in the production of M-CSF protein described herein.

M-CSF, as produced by the method of the present invention, is characterized by an apparent molecular weight of approximately 70 to 90kd when analyzed by polyacrylamide SDS gel electrophoresis under nonreducing conditions. However, if this analysis is performed after reduction of the M-CSF, the protein is characterized by an apparent molecular weight of 35 to 45kd, suggesting that M-CSF is a disulfide linked homo-dimer of 35 to 45kd subunits.

The approximately 61kd precursor encoded by the sequence of Fig. I is processed at the amino terminus by removal of a 32 residue signal peptide and in the carboxy terminal region by removal of about 229 residues to yield a subunit of approximately 223 or 224 amino acids with a predicted molecular weight of 21kd. Thus the mature M-CSF monomer has Glu at its amino terminal and extends at least through to amino acid Arg at position 223 in Fig. I. The protein may also contain the amino acid Ser at position 224 in Fig. I at its carboxy terminus. This subunit retains two of the four potential sites for addition of asparagine-linked carbohydrate that are present in the sequence of Fig. I. Glycosylation of the 21kd polypeptide at these two positions and at numerous O-linked glycosylation sites accounts for most of the remaining mass of the 35-45kd subunit of M-CSF.

As yet another aspect of the invention is a therapeutic composition for treating deficiencies in hematopoietic cells, particularly those cells of myeloid lineage, including monocytes and macrophages. M-CSF may be used to directly stimulate monocyte and macrophage production and may indirectly stimulate other hematopoietic lineages. Among conditions susceptible to treatment with the polypeptides of the present invention is leukopenia, a reduction in the number of circulating white cells in the peripheral blood. Leukopenia may be induced by exposure to viruses or to radiation, and is often a side effect of various cancer therapies and chemotherapeutic drugs. Treatment of leukopenia with M-CSF may avoid such side effects. In addition, M-CSF may activate mature white cells in the event of serious infection and cause monocytes to produce GM-CSF. M-CSF may also be employed to treat intracellular parasitic infections, bacterial infections and the like. Moreover, M-CSF alone, or in combination with other hematopoietins, such as IL-1, IL-2, IL-3, IL-4, IL-5, B-cell differentiation factor, BSF-2 and the interferons, may enhance macrophage function causing activated macrophages to kill tumor cells, release alpha-interferon, kill parasites or release and enhance other CSFs.

M-CSF may also be employed in the treatment of other blood cell deficiencies, including thrombocytopenia or anemia; in the treatment of patients recovering from bone marrow transplants; and to enhance host defense during surgery and in burn patients. M-CSF, in conjunction with an antibody [e.g., in the antibody dependent cell cytotoxicity method; see A. F. Lopez et al, J. Immunol., 131(6):2983-2988 (1983)] may activate tumor cell killing by human monocytes and thereby be an effective treatment for some cancers.

A therapeutic composition according to the present invention comprises a therapeutically effective amount of the M-CSF protein in admixture with a pharmaceutically acceptable carrier. This composition can be systemically administered either parenterally, intravenously or subcutaneously. When systemically administered, the therapeutic composition for use in this invention is, of course, in the form of a pyrogen-free, parenterally acceptable aqueous solution. The preparation of such a parenterally acceptable protein solution, having due regard to pH, isotonicity and stability, is within the skill of the art.

The dosage regimen will be determined by the attending physician considering various factors which modify the action of drugs, e.g. the condition, body weight, sex, and diet of the patient, the severity of any infection, time of administration and other clinical factors. Generally, the daily regimen should be in the range of 1 to 1000 micrograms of protein or 50 to 5000 units of protein (a unit being the concentration of polypeptide which leads to half maximal stimulation in a standard murine bone marrow assay) per kilogram of body weight. Progress can be monitored by periodic assessment of the hematological profile, e.g. white cell count and the like.

The therapeutic composition may also include co-administering other human factors with M-CSF. A non-exclusive list of other appropriate hematopoietins, CSFs and interleukins to interaction with the M-CSF of the invention includes: those factors listed above and GM-CSF, G-CSF, Meg-CSF, erythropoietin and eosinophil differentiation factor. The dosage recited above would be adjusted to compensate for such additional components in the therapeutic composition or method. M-CSF may also be administered in therapies employing monoclonal antibodies.

### Detailed Description of the Invention

The following examples illustrate the present invention employing the DNA sequence of Fig. I to produce M-CSF or naturally-occurring allelic variants (isozymes) thereof or deliberately modified M-CSF proteins.

### EXAMPLE I

### Construction of an exemplary mammalian expression vector p3ACSF-69

The complete DNA sequence of Fig. I was isolated from poly A+ mRNA of the SV40 transformed trophoblast cell line TPA-30-1 [ATCC #CRL-1583]. To construct a mammalian vector for expression of M-CSF, the complete cDNA sequence depicted in Fig. I above was adapted with restriction endonuclease enzyme XhoI linkers (New England Biolabs) and ligated into XhoI-digested, phosphatased COS cell expression vector pXM. pXM contains the SV40 enhancer, major adenovirus late promoter, DHFR coding sequence, SV40 late message poly-A addition site and VaI gene. pXM further contains a linker sequence with restriction endonuclease sites for KpnI, PstI and XhoI. The plasmid resulting from the XhoI digestion of pXM and the insertion of the linker and the XhoI adapted DNA sequence of Fig. I coding for M-CSF was designated p3ACSF-69. p3ACSF-69 (ATCC #67092) can be transformed by conventional techniques into a suitable mammalian host cell for expression of the M-CSF protein. Exemplary host cells are mammalian cells and cell lines, particularly primate cell lines, rodent cell lines and the like.

One skilled in the art can also construct other mammalian expression vectors comparable to p3ACSF-69 but containing less than the entire sequence of Fig. I. For example, the 5' and 3' flanking sequences may be cut from Fig. I, if desired; or modified or allelic variations of Fig. I may be employed by manipulating the sequence of Fig. I. The DNA sequence of Fig. I can be cut from the plasmid with XhoI and well-known recombinant genetic engineering techniques employed to modify the sequence and to insert it into other known vectors, such as pCD [Okayama et al., Mol. Cell Biol. 2:161-170 (1982)] and pJL3, pJL4 [Gough et al., EMBO J., 4:645-653 (1985)]. The transformation of these vectors into appropriate host cells can result in expression of M-CSF protein.

Similarly, one skilled in the art could manipulate the sequence by eliminating or replacing the mammalian regulatory sequences flanking the coding sequence with yeast, bacterial or insect sequences to create non-mammalian vectors. Thus this sequence would then be expressible in yeast, bacterial or insect host cells. For example, the coding sequence of Fig. I could be cut from p3ACSF-69 with XhoI and further manipulated (e.g. ligated to other known linkers or modified by deleting non-coding sequences therefrom or altering nucleotides therein by other known techniques). The modified M-CSF coding sequence could then be inserted into, for example, a known bacterial vector using procedures such as described in T. Taniguchi et al, Proc. Natl. Acad.Sci. U.S.A., 77: 5230-5233 (1980). This exemplary bacterial vector could then be transformed into bacterial host cells and M-CSF expressed thereby.

Similar manipulations can be performed for the construction of an insect vector [See, e.g. procedures described in published European patent application 155,476] or a yeast vector [See, e.g. procedures described in published PCT application WO 86 00639] for expression of the M-CSF protein in insect or yeast cells.

### EXAMPLE II

### Expression of M-CSF Protein

Plasmid DNA, prepared from E. coli HB101 containing p3ACSF-69 as described in Maniatis et al., Molecular Cloning, A Laboratory Manual, Cold Spring Harbor Laboratory, (1982) was purified by conventional methods involving equilibrium centrifugation in cesium chloride gradients containing ethidium bromide. COS cells (ATCC CRL 1650) were transfected with the purified DNA at a concentration of approximately 5µg plasmid DNA per 10⁶ COS cells and treated with chloroquine according to the procedures described in G. G. Wong et al., Science, 280: 810-815 (1985) and R. J. Kaufman et al. Mol. Cell Biol., 2:1304 (1982). 72 hours following transfection p3ACSF-69-containing COS cell conditioned medium can be harvested containing a protein which demonstrates M-CSF activity in standard bone marrow assays, as discussed in Example III below.

### EXAMPLE III

### M-CSF Activity in In Vitro Assays

### A. Mouse assay

Mouse bone marrow assays were conducted as described in D. Metcalf, The Hemopoietic Colony Stimulating Factors, Elsevier Press, New York (1984) with the following modifications:
(a) 2 x 10⁵ bone marrow cells per ml were employed in the assay;
(b) final assay volume was 100µl; and
(c) assays were set up in standard 96 well microtitre plates.

Bone marrow was obtained from the femurs of 6 - 25 week old female Balb/c mice (Jackson). Using WEHI 3 conditioned medium [J. C. Lee et al., J. Immunol., 128: 2393-2398 (1982)] which contains mouse L cell conditioned medium as a standard control, one dilution unit of activity was defined as that concentration of protein which results in a maximal response in this bone marrow assay, i.e. approximately 15 to 20 colonies per 2 x 10⁴ mouse bone marrow cells.

Conditioned medium from COS cells containing p3ACSF-69 was found to be active to at least 10⁻⁴ dilution in a mouse bone marrow assay and produced mainly monocytic lineage type colonies. The number and type of cells in a maximal response will vary with the strain and age of the mouse donors.

### B. Human Assay

Human bone marrow assays, employing non-adherent bone marrow cells, were performed as described in G. G. Wong, et al., supra. p3ACSF-69-containing COS cell conditioned medium was active to a 1:50 dilution in human bone marrow assays and produced predominantly monocytic lineage-type colonies.

### EXAMPLE IV

### Molecular Weight Analysis of M-CSF

Following the procedure of R. J. Kaufman and P. A. Sharp, J. Mol. Biol. 159:601-629 (1982), ³⁵S methionine can be metabolically incorporated into the M-CSF protein made by COS cell transfection with p3ACSF-69 DNA. When ³⁵S methionine labelled p3ACSF-69-containing COS cell conditioned medium is analyzed under non-reducing conditions by polyacrylamide gel electrophoresis, [U.K. Laemmli, Nature 227:680-685 (1970)] a broad band, indicative of glycosylation, can be detected at an apparent molecular weight of approximately 70kd. Corresponding transfection of pXM vector DNA does not produce an equivalent ³⁵S protein band.

³⁵S methionine labelled p3ACSF-69 conditioned medium was reduced with 100mM beta-mercaptoethanol and analyzed by SDS polyacrylamide gel electrophoresis. The 70kd band disappears and an approximately 35kd, broad band, indicative of glycosylation, is detected. Reduced p3ACSF-69-containing COS cell conditioned medium is inactive in both mouse and human bone marrow assays. Thus the M-CSF protein in the COS cell conditioned medium appears to be active as a dimer and inactive when it is reduced to its constitutive monomers.

³⁵S labelled, reduced p3ACSF-69-containing COS cell conditioned medium was treated with neuraminidase (Sigma) according to the procedures of M. Bradford, Anal. Biochem 72:248 (1976) to remove sialic acid and with peptide N--glycosidase F [also known as N-glycanase (Genzyme)] to remove N-linked carbohydrates. Analysis by SDS polyacrylamide gel electrophoresis revealed an approximately 18kd discrete band in the neuraminidase, N-glycosidase F treated p3ACSF-69-containing COS cell conditioned medium which was absent from the reduced untreated ³⁵S methionine labelled conditioned medium. Reduced ³⁵S labelled p3ACSF-69-containing COS cell conditioned medium treated only with N-glycanase revealed an approximately 18kd band in SDS-polyacrylamide gel electrophoretic analysis.

From N-glycosidase F and neuraminidase experiments, it appears that the M-CSF protein produced by COS cells transfected with p3ACSF-69 is a dimer of two approximately 35kd glycosylated monomers which each comprise an approximately 18kd polypeptide that is extensively modified by N-linked glycosylation.

### EXAMPLE V

### Construction of CHO cell lines expressing high levels of M-CSF

One method for producing high levels of M-CSF from mammalian cells involves the construction of cells containing multiple copies of the heterologous M-CSF gene. The heterologous gene can be linked to an amplifiable marker, e.g. the dihydrofolate reductase (DHFR) gene for which cells containing increased gene copies can be selected for by propagation in increasing concentrations of methotrexate (MTX) according to the procedures of Kaufman & Sharp, J.Mol. Biol., supra. This approach can be employed with a number of different cell types.

p3ACSF-69 and the DHFR expression plasmid pAdA26SV-(A)3 (Kaufman & Sharp, Mol. Cell Biol., supra) are cotransfected into DHFR-deficient CHO cells, DUKX-BII, by calcium phosphate coprecipitation and transfection. The initial DHFR expressing transformants are selected for growth in alpha media with dialyzed fetal calf serum, and subsequently selected for amplification by growth in increasing concentrations of MTX (sequential steps in 0.02, 0.2, 1.0 and 5mM MTX) as described in Kaufman, et al., Mol. Cell Biol. 5:1750 (1983).

One clone, designated CHO-3ACSF-69, which is selected for growth in 0.25 micromolar MTX was found to express high levels of biologically active M-CSF. This cell line consistently generates conditioned medium which is active in supporting murine macrophage colony formation at a 1:60,000 final dilution. These cells (one 10cm dish) as well as the parent CHO cells are labelled with 1 mCi of ³⁵S-met (NEN) in 4ml of Minimal Essential Medium (MEM) for 4 hours at 37C. The resulting conditioned media samples are incubated with antiserum raised in rabbits with purified urinary M-CSF. The antigen-antibody complexes are precipitated by adsorption to Staphylococcus aureus cells (Cal Biochem). The complexes are solubilized in a loading buffer lacking reducing agent according to U. K. Laemmli, Nature, 227:680-685 (1970). To reduce samples they are brought to 100mM 2-mercaptoethanol and incubated at 37C for 30 minutes. Following electrophoresis in 10% polyacrylamide gel, the patterns of labelled proteins are visualized by fluorography (Enhance, NEN) using Kodak XAR film.

Analysis of these immunoprecipitates by SDS polyacrylamide gel electrophoresis under non-reducing conditions revealed that the conditioned medium from the M-CSF protein-producing CHO cells contained two heterogeneous M-CSF protein species of apparent sizes 70-90kd and greater that 150kd. The observed size heterogeneity of these proteins is typical of many glycoproteins.

Analysis of the same samples following reduction revealed that the mobility of the 70-90kD species of M-CSF protein shifted to a position consistent with a molecular weight of 35-45kD while the relative mobility of the larger species (greater than 150kD) was unaffected by the treatment. Thus at least two different M-CSF proteins are expressed by the CHO-3ACSF-69 cells: a 70-90kD protein comprising a disulfide linked dimer of a 35-45kD subunit, and a much larger species. The cell lines referred to above have been adapted to serum-free, completely defined media.

### EXAMPLE VI

### Purification of M-CSF

The CHO-cell conditioned media containing .5% fetal bovine serum and DMEM-F12 is diluted 1:1 with water. The diluted media is then applied to a QAE 'Zeta-Prep' cartridge (LKB) which is equilibrated in 40mM Tris pH7.4. The flow-through containing unbound protein was discarded. Bound protein was washed with 40mM Tris, pH7.4 and eluted with 40mM Tris, pH7.4 and 0.75M NaCl. The eluate is then diluted with water to a concentration of 0.5M NaCl. Tween 20 was added to 0.05% and this mixture loaded at approximately 1 column volume/hour on to a lentil lectin-Sepharose 4B column [Pharmacia] which had been equilibrated in 20mM Tris, pH7.4, 0.5M NaCl and 0.05% Tween 20. The column was washed with 2 - 5cv, 20mM Tris, pH7.4, and 0.5M NaCl. Specifically-bound protein was eluted with 20mM Tris, 0.2M alphamethylmannopyranoside, 0.5M NaCl and 0.05% Tween 20, and then acidified with 10% trifluoroacetic acid [TFA]. The eluate was subjected to reverse phase liquid chromatography on a column equilibrated in 30% acetonitrile and 0.1% TFA. Protein was eluted with ascending acetonitrile in 0.1% TFA. Protein collected between 45 and 50% acetonitrile was neutralized in tubes with Tris, pH8.5 and analyzed.

The purification procedure also includes a size exclusion step to remove the high molecular weight aggregate. The preliminary analysis of M-CSF reveals a specific activity of approximately 10⁶ bone marrow units per milligram [see bone marrow assay in Example III].

Numerous modifications and variations in practice of this invention are expected to occur to those skilled in the art upon consideration of the foregoing descriptions of preferred embodiments thereof. Such modifications and variations are believed to be encompassed in the appended claims.

## Claims

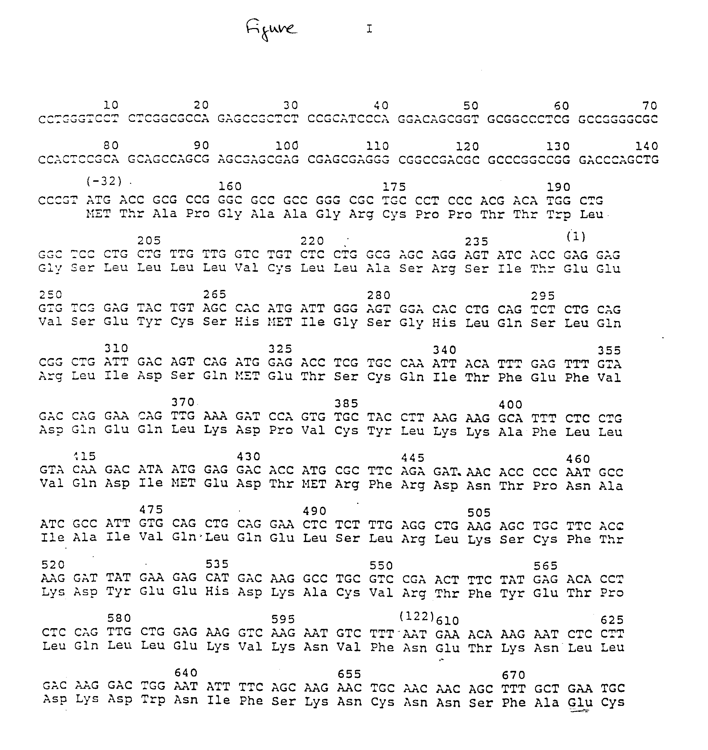

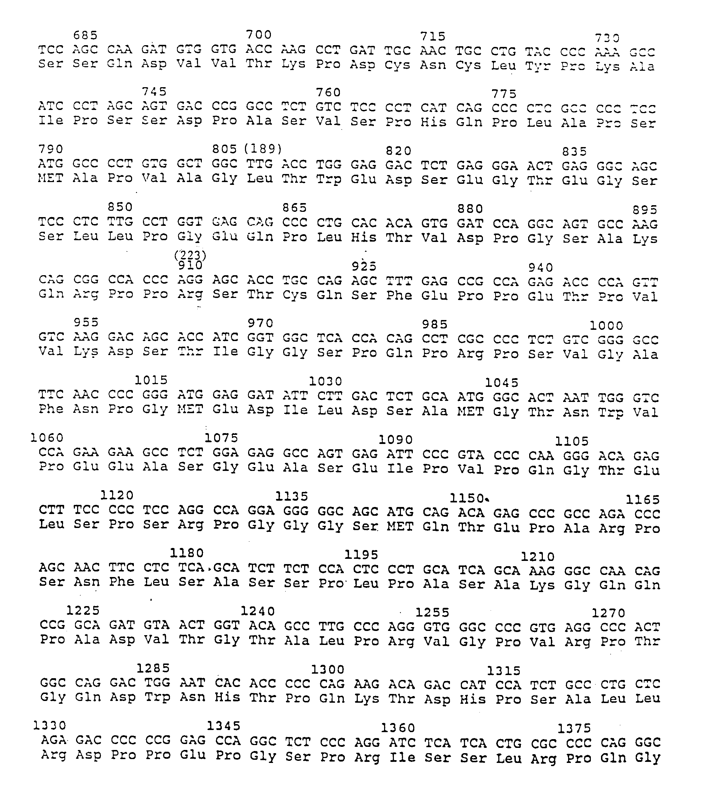

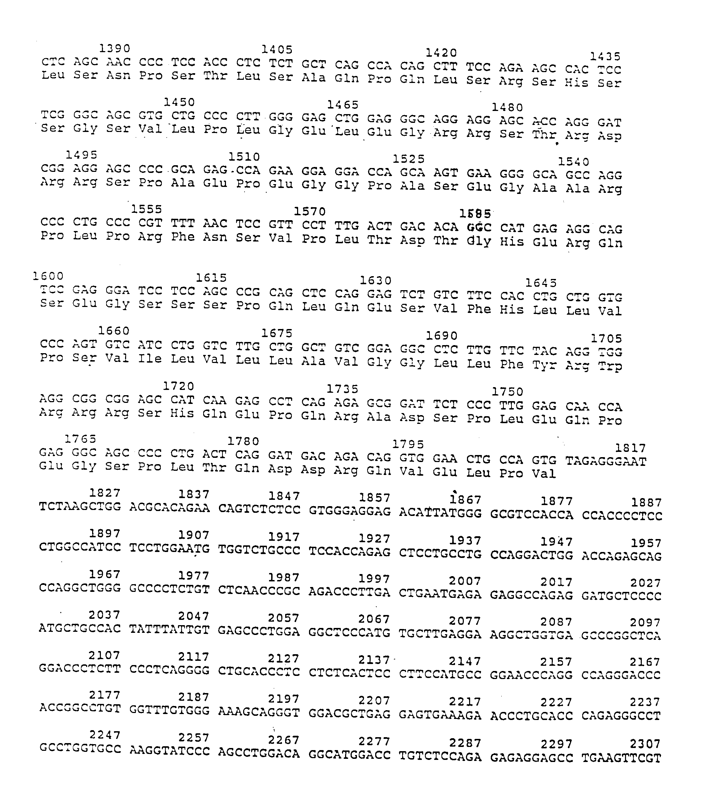

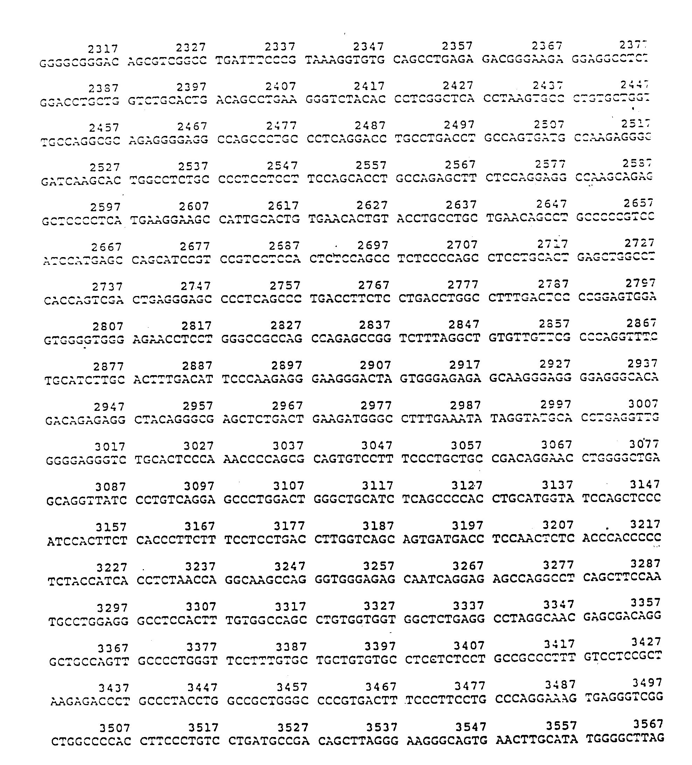

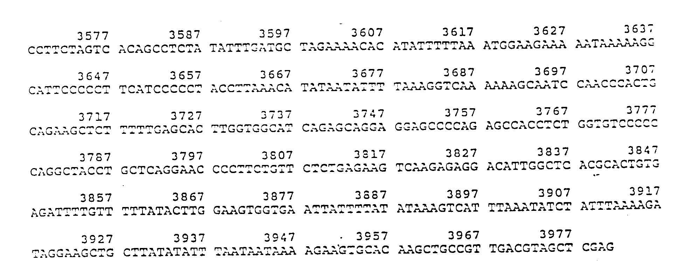

1. A DNA sequence encoding a polypeptide having M-CSF activity in mouse and human bone marrow assays comprising a nucleotide sequence selected from
(a) the nucleotide sequence corresponding to positions 689 to 910 of Figure 1;
(b) the nucleotide sequence corresponding to positions 242 to 910 of Figure 1;
(c) the nucleotide sequence corresponding to positions 146 to 1583 of Figure 1;
(d) the nucleotide sequence corresponding to positions 146 to 1807 of Figure 1;
(e) the complete nucleotide sequence of Figure 1; and
(f) a variant of any one of the nucleotide sequences of (a) to (e) resulting from
(i) allelic variations;
(ii) the degeneracy of the genetic code;
(iii) point mutations; or
(iv) nucleotide modifications.

2. The DNA sequence according to claim 1, which is the nucleotide sequence corresponding to positions 242 to 910 of Figure 1.

3. The DNA sequence according to claim 1 or 2, which is in operative association with an expression control sequence therefor.

4. A vector comprising a DNA sequence of any one of claims 1 to 3.

5. The vector according to claim 4, which is p3ACSF-69 (ATCC 67092).

6. A host cell, transformed with a DNA sequence according to any one of claims 1 to 3 or a vector according to claim 4 or 5.

7. The host cell according to claim 6, which is a mammalian cell, bacterial cell, yeast cell, or insect cell.

8. The host cell according to claim 6 or 7, which is E. coli HB 101 (ATCC 67092).

9. A process for producing a polypeptide having M-CSF activity, comprising culturing a host cell according to any one of claims 6 to 8.

10. A polypeptide having M-CSF activity, which is encoded by a DNA sequence according to any one of claims 1 to 3.

11. A therapeutic composition comprising an effective amount of the polypeptide according to claim 10.

12. The therapeutic composition according to claim 11 additionally containing a pharmaceutically acceptable carrier.

13. The therapeutic composition according to claim 11 or 12, further comprising an effective amount of at least one hematopoietic interleukin, growth factor or antibody.

14. The therapeutic composition according to any one of claims 11 to 13 for treating deficiencies in hematopoietic cells.

15. Use of a polypeptide according to claim 10 for the preparation of a pharmaceutical composition suitable for treating deficiencies in hematopoietic cells.

## Patentansprüche

1. DNA-Sequenz, die ein Polypeptid codiert mit M-CSF-Aktivität in Maus- und Mensch-Knochenmark-Tests, umfassend eine Nucleotidsequenz, ausgewählt aus
(a) der Nucleotidsequenz entsprechend den Positionen 689 bis 910 der Figur 1;
(b) der Nucleotidsequenz entsprechend den Positionen 242 bis 910 von Figur 1;
(c) der Nucleotidsequenz entsprechend den Positionen 146 bis 1583 von Figur 1;
(d) der Nucleotidsequenz entsprechend den Positionen 146 bis 1807 von Figur 1;
(e) der vollständigen Nucleotidsequenz von Figur 1; und
(f) einer Variante einer der Nucleotidsequenzen von (a) bis (e), die sich ergibt aus
(i) allelen Variationen;
(ii) der Degeneration des genetischen Codes;
(iii) Punktmutationen; oder
(iv) Nucleotidmodifikationen.

2. DNA-Sequenz nach Anspruch 1, die die Nucleotidsequenz entsprechend den Positionen 242 bis 910 von Figur 1 ist.

3. DNA-Sequenz nach Anspruch 1 oder 2, die in funktioneller Verbindung mit einer Expressionskontrollsequenz dafür steht.

4. Vektor, umfassend eine DNA-Sequenz nach einem der Ansprüche 1 bis 3.

5. Vektor nach Anspruch 4, der der Vektor p3ACSF-69 (ATCC 67092) ist.

6. Wirtszelle, die mit einer DNA-Sequenz nach einem der Ansprüche 1 bis 3 oder einem Vektor nach Anspruch 4 oder 5 transformiert ist.

7. Wirtszelle nach Anspruch 6, die eine Säugerzelle, eine Bakterienzelle, eine Hefezelle oder eine Insektenzelle ist.

8. Wirtszelle nach Anspruch 6 oder 7, die E. coli HB 101 (ATCC 67092) ist.

9. Verfahren zur Herstellung eines Polypeptids mit M-CSF-Aktivität, umfassend das Züchten einer Wirtszelle nach einem der Ansprüche 6 bis 8.

10. Polypeptid mit M-CSF-Aktivität, das von einer DNA-Sequenz nach einem der Ansprüche 1 bis 3 codiert wird.

11. Therapeutische Zusammensetzung, umfassend eine wirksame Menge des Polypeptids nach Anspruch 10.

12. Therapeutische Zusammensetzung nach Anspruch 11, die außerdem einen pharmazeutisch verträglichen Träger enthält.

13. Therapeutische Zusammensetzung nach Anspruch 11 oder 12, ferner umfassend eine wirksame Menge mindestens eines hämatopoetischen Interleukins, Wachstumsfaktors oder Antikörpers.

14. Therapeutische Zusammensetzung nach einem der Ansprüche 11 bis 13 zur Behandlung von Defiziten in hämatopoetischen Zellen.

15. Verwendung eines Polypeptids nach Anspruch 10 zur Herstellung eines Arzneimittels, das zur Behandlung von Defiziten in hämatopoetischen Zellen geeignet ist.

## Revendications

1. Séquence d'ADN codant pour un polypeptide ayant une activité de M-CSF dans des dosages de moëlle osseuse humaine et de souris, comprenant une séquence nucléotidique choisie parmi
(a) la séquence nucléotidique correspondant aux positions 689 à 910 de la figure 1 ;
(b) la séquence nucléotidique correspondant aux positions 242 à 910 de la figure 1 ;
(c) la séquence nucléotidique correspondant aux positions 146 à 1583 de la figure 1 ;
(d) la séquence nucléotidique correspondant aux positions 146 à 1807 de la figure 1 ;
(e) la séquence nucléotidique complète de la figure 1 ; et
(f) un variant d'une quelconque des séquences nucléotidiques de (a) à (e) provenant
(i) de variations alléliques ;
(ii) de dégénérescence du code génétique ;
(iii) de mutations ponctuelles ; ou
(iv) de modifications des nucléotides.

2. Séquence d'AN selon la revendication 1, qui est la séquence nucléotidique correspondant aux positions 242 à 910 de la figure 1.

3. Séquence d'AN selon la revendication 1 ou 2, qui est en association opérante avec une séquence de contrôle de l'expression pour celle-ci.

4. Vecteur comprenant une séquence d'ADN selon l'une quelconque des revendications 1 à 3.

5. Vecteur selon la revendication 4, qui est p3ACSF-69 (ATCC 67092).

6. Cellule hôte, transformée avec une séquence d'ADN selon l'une quelconque des revendications 1 à 3 ou un vecteur selon la revendication 4 ou 5.

7. Cellule hôte selon la revendication 6, qui est une cellule mammifère, une cellule bactérienne, une cellule de levure ou une cellule d'insecte.

8. Cellule hôte selon la revendication 6 ou 7, qui est E. coli HB 101 (ATCC 67092).

9. Procédé pour la production d'un polypeptide ayant une activité de M-CSF,qui comprend la culture d'une cellule hôte selon l'une quelconque des revendications 6 à 8.

10. Polypeptide ayant une activité de M-CSF, qui est codé par une séquence d'ADN selon l'une quelconque des revendications 1 à 3.

11. Composition thérapeutique comprenant une quantité efficace du polypeptide selon la revendication 10.

12. Composition thérapeutique selon la revendication 11, qui contient en plus un véhicule pharmaceutiquement acceptable.

13. Composition thérapeutique selon la revendication 11 ou 12,qui comprend en outre une quantité efficace d'au moins un anticorps, un facteur de croissance ou une interleukine hématopoïétique.

14. Composition thérapeutique selon l'une quelconque des revendications 11 à 13 pour le traitement des insuffisances dans les cellules hématopoïétiques.

15. Utilisation d'un polypeptide selon la revendication 10, pour la préparation d'une composition pharmaceutique, convenable pour traiter les insuffisances dans les cellules hématopoïétiques.
